# EUROPÄISCHE PATENTSCHRIFT

(11) **EP 2 364 526 B1**
(45) Veröffentlichungstag und Bekanntmachung des Hinweises auf die Patenterteilung: **09.10.2013**
(21) Anmeldenummer: 09804526.3
(22) Anmeldetag: 07.08.2009
(51) Int. Cl.: G06F 3/01, G06F 3/033, G06F 3/0354, G06F 3/038, H03K 17/955, A61B 5/11

(54) **SENSOREINRICHTUNG ZUR GENERIERUNG VON HINSICHTLICH DER POSITION ODER POSITIONSÄNDERUNG VON GLIEDMASSEN INDIKATIVEN SIGNALEN**
SENSOR DEVICE FOR GENERATING SIGNALS THAT ARE INDICATIVE OF THE POSITION OR CHANGE OF POSITION OF LIMBS
AGENCEMENT DE CAPTEURS DESTINÉ À PRODUIRE DES SIGNAUX INDICATIFS DE LA POSITION OU DU CHANGEMENT DE POSITION DE MEMBRES

(30) Priorität: 07.08.2008 DE 102008036720
(43) Veröffentlichungstag der Anmeldung: 14.09.2011
(73) Patentinhaber: Microchip Technology Germany II GmbH & Co. KG, 82205 Gliching (DE)
(72) Erfinder: IVANOV, Artem, 82205 Gilching (DE)
(74) Vertreter: Grubert, Andreas
(86) Internationale Anmeldenummer: PCT/EP2009/005754
(87) Internationale Veröffentlichungsnummer: WO 2010/015417

(56) Entgegenhaltungen:
- EP-A1- 1 593 988
- WO-A2-2008/009687
- DE-U1-202007 017 303

## Beschreibung

Die Erfindung bezieht sich auf eine Sensoreinrichtung gemäß dem Oberbegriff von Patentanspruch 1.

Diese Sensoreinrichtung dient der Generierung von elektronischen Signalen die als solche Aufschluss über eine räumliche Position, und/oder die Bewegung von Gliedmaßen, insbesondere der Hand eines Anwenders gegenüber der Sensoreinrichtung geben. Diese elektronischen Signale können dann zur Abwicklung von Eingabevorgängen bei Datenverarbeitungs-, Kommunikations- und anderweitigen Elektrogeräten herangezogen werden. Die erfindungsgemäße Sensoreinrichtung arbeitet insoweit als Man/Machine-Interface.

Die Erfindung richtet sich dabei im engeren Rahmen auf ein Gestenerfassungssystem zur berührungslosen Erfassung von Gesten, insbesondere Hand- oder Fingergesten auf kapazitivem Wege. Insbesondere richtet sich die Erfindung hierbei auf ein Gestenerfassungssystem mit vereinfachter Integrationsmöglichkeit in elektronische Geräte. Die Erfindung zielt hierbei darauf ab ein hinsichtlich seiner Funktion besonders zuverlässiges, relativ hochauflösendes Gesten-Interface für Steuerungszwecke insbesondere von Kommunikationsgeräten, sowie im Bereich von Bedienblenden und Cockpitsystemen zu realisieren.

Aus DE 20 2007 017303 U1 ist eine Sensoreinrichtung der eingangs genannten Art bekannt. Diese Sensoreinrichtung ist in eine Computermaus eingebunden und umfasst mehrere Elektroden die eine berührungslose Erfassung der Position der Hand eines Anwenders auf feldelektrischem Wege ermöglichen.

Aus WO 2008/009687 ist ein Dünn-Filmdrucksensor bekannt. Die Signalerfassung erfolgt hier durch physische Kontaktierung einer Eingabefläche. Eine berührungslose Positionserfassung ist gemäß diesem Stand der Technik nicht möglich.

Der Erfindung liegt in die Aufgabe zugrunde, Lösungen zu schaffen durch welche hinsichtlich der Position und/oder der Bewegung von Gliedmaßen indikative Signale in besonders vorteilhafter Weise generiert werden können.

Diese Aufgabe wird erfindungsgemäß gelöst durch eine Sensoreinrichtung gemäß Patentanspruch 1.

Dadurch wird es auf vorteilhafte Weise möglich, die zur Generierung des Geberelektrodenfeldes, sowie den zur Auswertung der durch die Sensorelektroden erfassten Spannungen vorgesehene elektronische Schaltung äußerst nahe an den Elektroden anzuordnen, ohne dass hierbei eine die Messsignale verfälschende Feldkoppelung zwischen den Sensorelektroden und der elektronischen Schaltung erfolgt.

Das erfindungsgemäße unter Verwendung einer typischerweise zentralen Geberelektrode und wenigstens drei hierum verteilt angeordneten Empfangselektroden gebildete "Kleeblatt"-System bietet eine für einen typischen Handbewegungsraum und damit für viele Anwendungen passende Reichweite und Genauigkeit bei der Erfassung der Gesteninformation.

Durch das erfindungsgemäße Konzept ergeben sich erheblich verminderte Auswirkungen des Erdschlusses etwaiger Schaltungskomponenten im Bereich der Messschaltung, sowie auch verminderte Interferenzen zwischen Schaltkreisen des anzusteuernden Gerätes an sich.

Die Reichweite eines kapazitiven Erfassungssystems wird im wesentlichen durch den Raum festgelegt, in welchem das zu Detektionszwecken aufgebaute elektrische Feld mit hinreichender (relativer) Stärke vorhanden ist. Durch das erfindungsgemäße Konzept wird in vorteilhafter Weise der Effekt reduziert, dass die an Erde gekoppelten Objekte in der Nähe von der feldgebenden Elektrode, d.h. der Feldbereitungselektrode des Erfassungssystems bewirken, das das elektrische Feld sich stark in den Raum zwischen der Elektrode und dem Objekt konzentriert. Dementsprechend wird durch das erfindungsgemäße Konzept der erfassbare Bereich vergrößert. Insbesondere in Verbindung mit dem Einsatz einer Transimpedanzbeschaltung der Messelektroden wird der Einfluss eines hier ansonsten noch vorhanden virtuellen Erdpotentials signifikant reduziert.

Die bei dem erfindungsgemäßen Erfassungssystem vorgesehene Elektrodengruppe ("Kleeblatt") ist an sich vorzugsweise nur mit einer möglichst geringen Erdverbindung ausgeführt. Die ggf. erdverbundenen Zuleitungen sind vorzugsweise abgeschirmt. Die Messelektroden sind vorzugsweise hochohmig an die Messschaltung angeschlossen, um die Feldverbreitung nicht zu stören.

Erfindungsgemäß wird das Messsystem derart ausgebildet, dass der Einfluss der im Bereich des Messsystems vorhandenen erdverbundenen Teile (insbesondere die eigene Elektronik) von der Geberelektrode sowie den hierum verteilt angeordneten Messelektroden abgeschirmt ist und/oder eine Feldüberbrückung kompensiert wird.

Die erste, die zweite und die dritte Empfangselektrodeneinrichtung jeweils an hochohmige Eingänge eines Impedanzwandlersystems angebunden, wobei anhand von Unterschieden zwischen den and den Ausgängen des jeweiligen Impedanzwandlers anliegenden elektrischen Ereignissen die orts- oder bewegungsindikativen Informationen gewonnen werden.

Aus den von den Empfangselektroden abgegriffenen Pegeln kann ein Summensignal gebildet werden, das die Summe der an den Empfangselektroden anliegenden Spannungen abbildet. Dieses Summensignal kann mit einem vorgegebenen Verstärkungsfaktor normiert werden und dem einem Komparatoreingang des jeweiligen Impedanzwandlersystems zur Verfügung gestellt werden.

Vorzugsweise ist ein Synchrondetektorystem vorgesehen, das als solches hinsichtlich der Synchronität des AusgangssignaJs am jeweiligen Impedanzwandlersystem, insbesondere bezüglich des Spannungspegels und/oder der Phase gegenüber der Erregerspannung indikative Signale liefert. Anhand von Unterschieden zwischen den and den Ausgängen des jeweiligen Synchrondetektors anliegenden elektrischen Ereignissen die orts- oder bewegungsindikativen Informationen gewonnen werden

Gemäß einer besonders bevorzugten Ausführungsform der Erfindung sind die Empfangselektroden symmetrisch um die Geberelektrodeneinrichtung herum angeordnet. Die Geberelektrode wird vorzugsweise von einem Generator (Mikrocontroller) mit Wechselspannung versorgt und um sich herum ein elektrisches, an sich vorzugsweise quasistatisches elektrisches Feld zu bilden. Die Empfangselektroden sind vorzugsweise symmetrisch um die Geberelektrode angeordnet.

Die an den Empfangselektroden anliegende elektrische Spannung beinhaltet Information über die Verteilung des Feldes der Geberelektrode.

Die Empfangselektroden sind an die hochohmigen Eingänge der Impedanzwandler angeschlossen um das elektrische Feld der Geberelektrode nicht zu stören. Aus den jeweiligen Signalen nach dem Impedanzwandler wird ein Mittelwert gebildet. Dieser Mittelwert kann verstärkt werden. Aus den verstärkten Signalen kann mit Hilfe eines Synchrondetektors die Amplitude gewonnen werden und weiter von einem ADC digitalisiert und für Auswertung in einen Mikrocontroller übergeben werden. Statt des Synchrondetektors kann auch ein ein Diodengleichrichter oder Peakdetektor verwendet werden.

Die erfindungsgemäße Sensoreinrichtung kann insbesondere der Erfassung von Gesten, Bewegungen oder Hand-Position dienen. Vorzugsweise ist zumindest der überwiegende Teil der Sensorelektronik inklusive Messelektroden in einer kompakten chip-ähnlichen Form aufgebaut. Hierdurch wird eine besonders vorteilhafte Anwendung der Sensortechnik ermöglicht.

Durch das erfindungsgemäße Konzept wird es auf vorteilhafte Weise möglich, große Reichweite des Detektors für Gesten zu erreichen, wobei der Detektor kompakt aufgebaut werden kann. Durch das erfindungsgemäße Konzept lässt sich bei Detektorabmessung von 3,5cm eine Reichweite von etwa 20cm erreichen. Bei der erfindungsgemäßen Schaltung sind die Eingänge hochohmig ausgelegt. Hochohmige Eingänge tragen in überraschend wirkungsvoller Weise zur Vergrößerung der Reichweite bei. Dies kann insbesondere durch eine Feldsimulationen visualisiert und insgesamt zuverlässig experimentell nachgewiesen werden. Erfindungsgemäß wird für jeden Kanal die Differenz seines Signals und des Mittelwertes verstärkt. Dies ermöglicht es bei symmetrischen Elektroden oder bei in der Elektronik symmetrisierten Signalen einen hohen Dynamikbereich auch mit einfachen ADCs (z.B. 10bit) zu erreichen. Dadurch werden mit einfachen Mitteln sehr kleine Signaländerungen erfassbar. Die Abschirmelektrode kann auch mit einer Spannung beaufschlagt werden die im wesentlichen die gleiche Amplitude aufweist wie die an der Geberelektrode anliegende Spannung. Die Schaltung kann so ausgeführt sein, dass diese eine relativ große Anzahl von Elektroden, insbesondere 8 Elektroden bietet, d.h. es werden 8 Kanäle realisiert. Außer einer Signalkompensierung durch Summenbildung ist auch eine Kompensierung durch Abziehen der gewichteten Generatorsignale möglich. Dadurch müssen die Elektroden nicht mehr gleich groß sein und nicht mehr symmetrisch angeordnet sein. Die Verstärker können auch als Linearverstärker ausgeführt sein. Zur Abschirmung kann direkt das Generatorsignal genommen werden, d.h. nicht zwingend ein Signal, das sich nur in der Amplitude von ihm unterscheidet.

Weitere Einzelheiten und Merkmale der Erfindung ergeben sich aus der nachfolgenden Beschreibung in Verbindung mit der Zeichnung. Es zeigt:
- **Figur 1**: ein Blockschaltbild zur Veranschaulichung des Grundaufbaus einer erfindungsgemäßen Sensoreinrichtung;
- **Figur 2**: eine Schemadarstellung zur Veranschaulichung eines beispielhaften Aufbaus der Geber- und der Empfangselektrodeneinrichtungen;
- **Figur 3**: eine Skizze zur Veranschaulichung einer beispielhaften Anordnung der Geber- und der Empfangselektrodeneinrichtungen bei einer als Gesteninterface ausgebildeten Computermaus;
- **Figur 4**: eine weitere Skizze zur Veranschaulichung einer beispielhaften Anordnung der Geber- und der Empfangselektrodeneinrichtungen bei einem mobilen Kommunikationsgerät.

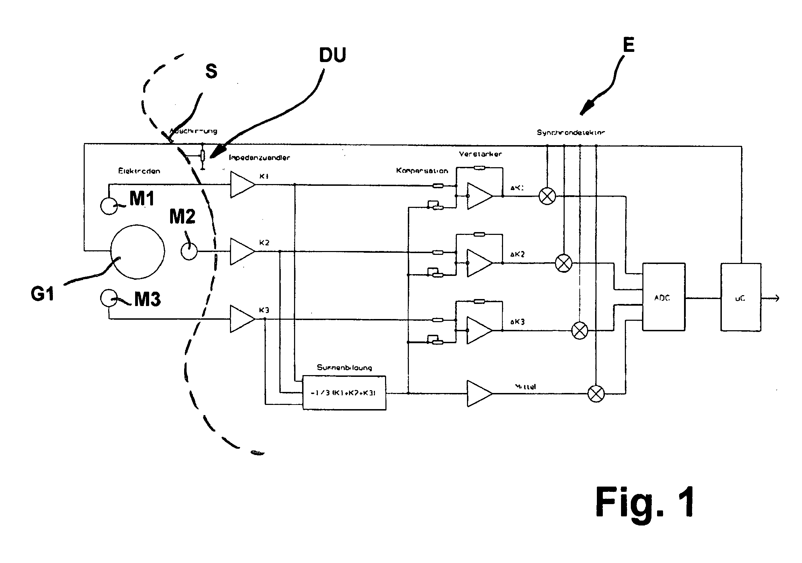

Wie aus dem in Figur 1 dargestellten Schaltungsplan ersichtlich kann das erfindungsgemäße System mit einer Flächeelektrodengruppe realisiert werden die gegenüber der eigentlichen Mess- und Treiberschaltung in der dargestellten Weise abgeschirmt ist.

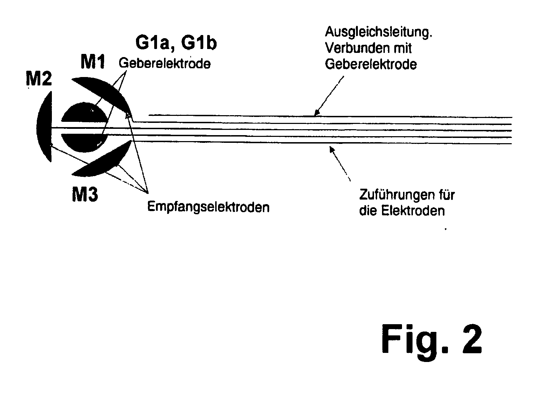

Die Elektroden befinden sich räumlich von der restlichen Elektronik abgegrenzt. Sie können z.B. auf einer Leiterplatte als Kupferflächen ausgebildet sein oder als leitende Bereiche an einer Kunststoff-Folie ausgeführt sein, die an die Elektronik angeschlossen wird. Die Messelektroden M1, M2, M3 (2, 3, 4 oder mehr) sind symmetrisch um die Geberelektrode G1 angeordnet (vgl. Beispiel für 3 Elektroden in Fig. 2). Die Elektroden M1, M2, M3, G1 werden über dünne Leiterbahnen mit der Elektronik E verbunden, so dass der Einfluss der Verbindungen möglichst klein im Vergleich zu Elektroden ist. Die Zuführungen können insbesondere durch z.B. mehrlagigen Aufbau zusätzlich abgeschirmt sein.

Der feldstörende Einfluss der Elektronik des Erfassungssystems und des Gerätes wird durch eine Abschirmung S minimiert. Die Abschirmelektrode S grenzt das Elektrodensystem M1, M2, M3, G1 von der Elektronik E ab. Diese Elektrode S kann z.B. aus einer Kunststofffolie mit leitender Beschichtung gefertigt sein.

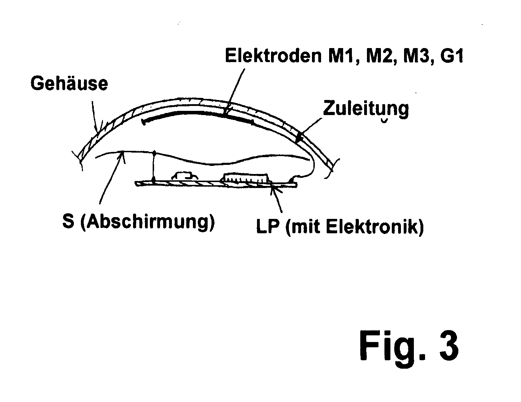

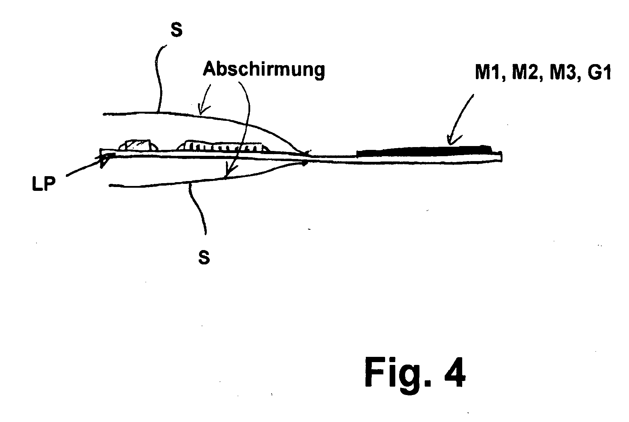

Beispiele der räumlichen Ausführung sind in Fig. 3 und Fig. 4 dargestellt. Die Elektroden in Fig. 3 sind auf einer Kunststofffolie über der Leiterplatte LP angebracht.

Bei dem Aufbau nach Figur 4 sind die Elektroden M1, M2, M3, G1 als Kupferflächenabschnitte in die Leiterplatte integriert.

Die Abschirmelektrode S wird mit einem Signal beaufschlagt, das sich nur in der Amplitude vom Signal auf der Geberelektrode G1 unterscheidet. Diese Amplitude ist so gewählt, dass die hinter der Abschirmelektrode S "verborgene" Elektronik vom elektrischen Feld der Geberelektrode G1 "getarnt" ist. Das heißt, das Potential an der Abschirmelektrode S entspricht dem von der Geberelektrode G1 erzeugten Potenzial an der gleicher Stelle im Falle dass die Elektronik und die Abschirmelektrode nicht präsent wären. Das Signal für die Abschirmelektrode kann z.B. mit Hilfe eines (einstellbaren) Spannungsteilers DU aus dem Signal der Geberelektrode G erzeugt werden.

Die unvermeidbaren quasistatischen Differenzen der Signale der Empfangselektroden M1, M2, M3 werden in der Schaltung elektronisch kompensiert. Dafür wird vorgesehen, dass das gebildete Summensignal mit unterschiedlicher Gewichtung an die Verstärker einzelner Kanäle zugeführt wird (Fig. 1). Die Gewichtung wird für jeden Kanal angepasst, um die Ausgangssignale der Verstärker im Ursprungszustand des Messsystems möglichst nahe bei Null zu halten. Unter dieser Bedingung wird mit den (nichtlinearen) Verstärkern die beste Empfindlichkeit für die Änderungen der Eingangssignale erreicht.

Die Wirksamkeit der Kompensation wird verbessert wenn die Empfangselektroden möglichst gleiche Kopplung zur Geberelektrode aufweisen. Diese Kopplung kann z.B. durch zusätzliche Elektroden beeinflusst werden:

In Fig. 2 ist gezeigt, wie eine zusätzliche "Ausgleichsleitung" die Kopplung von der Elektrode M1 zu einer geteilten Geberelektrode G1a, G1b erhöht und zwar auf den Pegel der Kopplung der Elektroden M2 und M3.

Die Kompensation kann auch während des Betriebs dynamisch nachgestellt werden, um das Messsystem auf sich eventuell ändernde Einflüsse abzustimmen.

Durch das erfindungsgemäße Konzept wird es möglich, die Gestenerfassungselektronik besonders vorteilhaft unmittelbar in elektronische Geräte, z.B. Mobiltelefone zu integrieren. Weiterhin wird es möglich, die Elektroden unmittelbar in die Leiterplatte zu integrieren, oder als leitende Schicht auf einer Kunststofffolie auszuführen. Zudem wird es möglich, die Anbindung der Elektroden an das Messsystem ohne Koaxialkabel vorzunehmen. Durch das erfindungsgemäße Konzept wird weiterhin eine Maximierung der Reichweite des Erfassungssystems bei gegebenen Umgebungseigenschaften erreicht. In vorteilhafter Weise ergibt sich auch eine Linearisierung der Systemantwort auf die Bewegung des zu detektierenden Objektes.

## Patentansprüche

1. Sensoreinrichtung zur Generierung von elektrischen Signalen die als solche Aufschluss über die Position oder Bewegung von Gliedmaßen gegenüber einem Bezugsbereich geben, mit:
- einer Geberelektrodeneinrichtung (G1, G1a, G1b),
- einem Spannungsgenerator, zur Beaufschlagung der Geberelektrodeneinrichtung (G1, G1a, G1b) mit einer Wechselspannung;
- einer ersten Empfangselekrodeneinrichtung (M1),
- einer zweiten Empfangselektrodeneinrichtung (M2), und
- einer Messschaltung zur Erfassung eines mit einer Hand- oder Fingergeste korrelierenden Feldüberbrückungseffektes zu den Empfangselektrodeneinrichtungen (M1, M2),
**dadurch gekennzeichnet,**
- **dass** eine Abschirmungselektrodeneinrichtung (S) vorgesehen ist die als solche die Geberelektrodeneinrichtung (G1, G1a, G1b) und/oder die Empfangselektrodeneinrichtungen (M1, M2) gegenüber der Messschaltung feldelektrisch zumindest weitgehend abschottet, und dass die erste, und die zweite Empfangselektrodeneinrichtung jeweils an hochohmige Abgriffssysteme angebunden sind, und
- **dass** die Abschirmelektrode S mit einem Signal beaufschlagt wird, das sich nur in der Amplitude vom Signal auf der Geberelektrode G1 unterscheidet

2. Sensoreinrichtung nach Anspruch 1, **dadurch gekennzeichnet, dass** die Empfangselektrodeneinrichtung eine dritte Empfangselektrodeneinrichtung (M3) umfasst.

3. Sensoreinrichtung nach Anspruch 1 oder 2, **dadurch gekennzeichnet, dass** die dritte Empfangselektrodeneinrichtung an ein hochohmiges Abgriffssystem angebunden ist.

4. Sensoreinrichtung nach Anspruch 1, **dadurch gekennzeichnet, dass** diese Amplitude ist so eingestellt wird, dass die hinter der Abschirmelektrode S "verborgene" Elektronik vom elektrischen Feld der Geberelektrode G1 "getarnt" ist.

5. Sensoreinrichtung nach Anspruch 4, **dadurch gekennzeichnet, dass** der Pegel im wesentlichen so eingestellt wird, dass das Potential an der Abschirmelektrode S dem von der Geberelektrode G1 erzeugten Potenzial an der gleicher Stelle im Falle dass die Elektronik und die Abschirmelektrode nicht präsent wären, entspricht.

6. Sensoreinrichtung nach Anspruch 5, **dadurch gekennzeichnet, dass** das Signal für die Abschirmelektrode mit Hilfe eines dynamisch einstellbaren Spannungsteilers DU aus dem Signal der Geberelektrode G erzeugt wird.

7. Sensoreinrichtung nach wenigstens einem der Ansprüche 1 bis 6, **dadurch gekennzeichnet, dass** auftretende quasistatische Differenzen der Signale der Empfangselektroden M1, M2, M3 in der Schaltung elektronisch kompensiert werden.

8. Sensoreinrichtung nach Anspruch 7, **dadurch gekennzeichnet, dass** hierzu ein Summensignal gebildet wird und mit unterschiedlicher Gewichtung an die Verstärker einzelner Kanäle zugeführt wird

9. Sensoreinrichtung nach Anspruch 8, **dadurch gekennzeichnet, dass** die Gewichtung für jeden Kanal angepasst wird, um die Ausgangssignale der Verstärker im Ursprungszustand des Messsystems möglichst nahe bei Null zu halten.

## Claims

1. A sensor device for generating electric signals, which as such give information on the position or movement of limbs with respect to a reference area, comprising:
- a pickup electrode device (G1, G1a, G1b),
- a voltage generator for applying an alternating voltage to the pickup electrode device (G1, G1a, G1b);
- a first receiving electrode device (M1),
- a second receiving electrode device (M2), and
- a measurement circuit for detecting a field bridging effect to the receiving electrode devices (M1, M2) that correlates with a gesture of a hand or a finger,
**characterized in that**
- a shielding electrode device (S) is provided, which as such, at least to a large extent, field-electrically seals off the pickup electrode device (G1, G1a, G1b) and/or the receiving electrode devices (M1, M2) from the measurement circuit, and the first and the second receiving electrode device each are connected to high-resistance tapping systems, and
- the shielding electrode S is loaded with a signal, which only differs from the signal at the pickup electrode G1 in its amplitude.

2. The sensor device of claim 1, **characterized in that** the receiving electrode device comprises a third receiving electrode device (M3).

3. The sensor device of claim 1 or 2, **characterized in that** the third receiving electrode device is connected to a highly resistive tapping system.

4. The sensor device of claim 1, **characterized in that** this amplitude is adjusted such that the electronics "hidden" behind the shielding electrode S is "disguised" by the electric field of the pickup electrode G1.

5. The sensor device of claim 4, **characterized in that** the level substantially is adjusted such that the potential at the shielding electrode S corresponds to the potential generated by the pickup electrode G1 at the same place in the case that the electronics and the shielding electrode would not be present.

6. The sensor device of claim 5, **characterized in that** the signal for the shielding electrode is generated from the signal of the pickup electrode G with the help of a dynamically adjustable potential divider DU.

7. The sensor device of at least one of claims 1 to 6, **characterized in that** emerging quasi-static differences of the signals of the receiving electrodes M1, M2, M3 are electronically compensated in the circuit.

8. The sensor device of claim 7, **characterized in that** a sum of signals is formed and is fed to the amplifiers of individual channels with differing weighting.

9. The sensor device of claim 8, **characterized in that** the weighting is adapted for each channel to keep the output signals of the amplifiers as close to zero as possible in the originating state of the measurement system.

## Revendications

1. Dispositif de capteurs destiné à générer des signaux électriques qui, en tant que tels, donnent des indications sur la position ou le mouvement des membres par rapport à une zone de référence, comportant :
- un dispositif d'électrodes d'émission (G1, G1a, G1b),
- un générateur de tension, destiné à alimenter le dispositif d'électrodes d'émission (G1, G1a, G1b) par une tension alternative,
- un premier dispositif d'électrodes de réception (M1),
- un deuxième dispositif d'électrodes de réception (M2), et
- un circuit de mesure pour détecter un effet de shuntage du champ, en corrélation avec un geste de la main ou du doigt, par rapport aux dispositifs d'électrodes de réception (M1, M2),
**caractérisé**
- **en ce qu'** un dispositif d'électrodes de protection (S) est prévu qui, en tant que tel, isole au moins en grande partie, sur le plan du champ électrique, le dispositif d'électrodes d'émission (G1, G1a, G1b) et/ou les dispositifs d'électrodes de réception (M1, M2) par rapport au circuit de mesure, et en ce que le premier et le deuxième dispositif d'électrodes de réception sont raccordés, respectivement, à des systèmes de prises à haute impédance, et
- **en ce que** l'électrode de protection (S) reçoit un signal qui se différencie de celui de l'électrode d'émission (G1) uniquement par son amplitude.

2. Système de capteurs selon la revendication 1, **caractérisé en ce que** le dispositif d'électrodes de réception comprend un troisième dispositif d'électrodes de réception (M3).

3. Système de capteurs selon la revendication 1 ou 2, **caractérisé en ce que** le troisième dispositif d'électrodes de réception est raccordé à un système de prises à haute impédance.

4. Système de capteurs selon la revendication 1, **caractérisé en ce que** cette amplitude est réglée de telle sorte que le système électronique « cachée » derrière l'électrode de protection (S) est « masquée » par le champ électrique de l'électrode d'émission (G1).

5. Système de capteurs selon la revendication 4, **caractérisé en ce que** le niveau est réglé sensiblement de telle sorte que le potentiel au niveau de l'électrode de protection (S) correspond au potentiel généré par l'électrode d'émission (G1) au même emplacement dans le cas où le système électronique et l'électrode de protection ne seraient pas présents.

6. Système de capteurs selon la revendication 5, **caractérisé en ce que** le signal pour l'électrode de protection est généré à partir du signal de l'électrode d'émission (G) au moyen d'un diviseur de tension (DU), réglable sur le plan dynamique.

7. Système de capteurs selon au moins l'une quelconque des revendications 1 à 6, **caractérisé en ce que** des différences quasi-statiques apparaissant au niveau des signaux des électrodes de réception (M1, M2, M3) sont compensées électroniquement dans le circuit.

8. Système de capteurs selon la revendication 7, **caractérisé en ce qu'**un signal composite est formé à cet effet et est acheminé avec une pondération différente vers les amplificateurs de différents canaux.

9. Système de capteurs selon la revendication 8, **caractérisé en ce que** la pondération est adaptée pour chaque canal, afin de maintenir autant que possible au voisinage de zéro les signaux de sortie des amplificateurs à l'état initial du système de mesure.
